# EUROPEAN PATENT SPECIFICATION

(11) **EP 3 236 741 B1**
(45) Date of publication and mention of the grant of the patent: **06.03.2019**
(21) Application number: 15830853.6
(22) Date of filing: 21.12.2015
(51) Int. Cl.: G01N 33/00, A01G 9/00

(54) **MICROCOSM FOR PLANT GROWTH**
MIKROKOSMOS FÜR PFLANZENWACHSTUM
MICROCOSME POUR LA CROISSANCE DES PLANTES

(30) Priority: 22.12.2014 IT RM20140743
(43) Date of publication of application: 01.11.2017
(73) Proprietor: ENEA - Agenzia Nazionale per le Nuove Tecnologie, l'Energia e lo Sviluppo Economico Sostenibile, 00196 Roma (RM) (IT); FOS S.r.l., 20123 Milano (IT); Sesmat S.r.l., 82018 San Giorgio del Sannio (BN) (IT)
(72) Inventor: D'AQUINO, Luigi, 00196 Roma (IT); MINARINI, Carla, 00196 Roma (IT); LANZA, Bruno, 00196 Roma (IT); ATRIGNA, Mauro, 00196 Roma (IT); DE FILIPPO, Giovanni, 00196 Roma (IT); PANDOLFI, Giuseppe, 00196 Roma (IT); GIANNOTTA, Giovanni, 39100 Bolzano (IT); PEDICINI, Antonio, 82030 Foglianise (BN) (IT)
(74) Representative: Sarpi, Maurizio
(86) International application number: PCT/IB2015/059835
(87) International publication number: WO 2016/103152

(56) References cited:
- EP-A2- 1 074 838
- CN-U- 201 662 570
- DE-A1- 19 905 734
- RU-C1- 2 038 747

## Description

The present invention basically relates to a microcosm for growing plants under biotic and abiotic conditioning, constituted by equipment devised, designed, and produced to enable growing of terrestrial plant organisms in a confined environment and in given environmental conditions. Such a microcosm is disclosed in DE19905734. The invention finds application in tests that involve interaction between plant and environment. The microcosm is an environment in which it is possible to monitor, i.e., control in a constant, continuous, and methodical way, and manage different micro-environmental conditions diversified between the part that is designed for housing the epigean part and the part that is designed for housing the hypogean part of the plants. Inside the microcosm, it is also possible to grow organisms of different nature in the cultivation environment of the plant, limiting the diffusion of symbionts, pathogens, and parasites both from the external environment and towards the external environment. Modularity, sectionability and accessibility enable the apparatus to be utilized in many different research applications. The reduced dimensions enable the apparatus to be placed in environments with small extensions. The characteristics of construction enable it to be located both in indoor and outdoor environments. The requirements in terms of power consumption enable location of the microcosm in a common research laboratory. The equipment is designed so as to be able to house sensor systems for monitoring various micro-environmental parameters. The system for managing the environmental parameters enables remote management of all the functions, thus making possible placing the apparatus also in less accessible environments.

A better understanding of the invention will be obtained from the following detailed description and with reference to the attached drawings, which illustrate a preferred embodiment thereof purely by way of example.

In the drawings:
Figure 1 is an overview of the principal factors affecting the plant functions;
Figure 2 represents a microcosm of a known type;
Figure 3 represents cylinders and funnels of a known type for growing the radical part of the plants of a microcosm;
Figure 4 is a front-side overview of the microcosm as a whole;
Figure 5 is a front-side overview of the microcosm as a whole from the side opposite to that of the previous figure;
Figure 6 is a front-side view of the microcosm as a whole, with the doors of the hypogeal chamber open to show the system of cylinders and funnels for growing the radical part of the plants;
Figure 7 shows a longitudinal section of the microcosm as a whole bearing the arrangement of cylinders and funnels for raising the radical part of the plants in a front-side view;
Figure 8 shows a longitudinal section of the microcosm as a whole bearing the arrangement of cylinders and funnels for growing the radical part of the plants in a front view;
Figure 9 represents the guide shelves for housing the cylinders and funnels, as well as a cylinder and a funnel; and
Figure 10 represents the hypogean chamber of the microcosm carrying the arrangement of boxes and saucers, as well as a frusto-pyramidal box and the corresponding saucer.

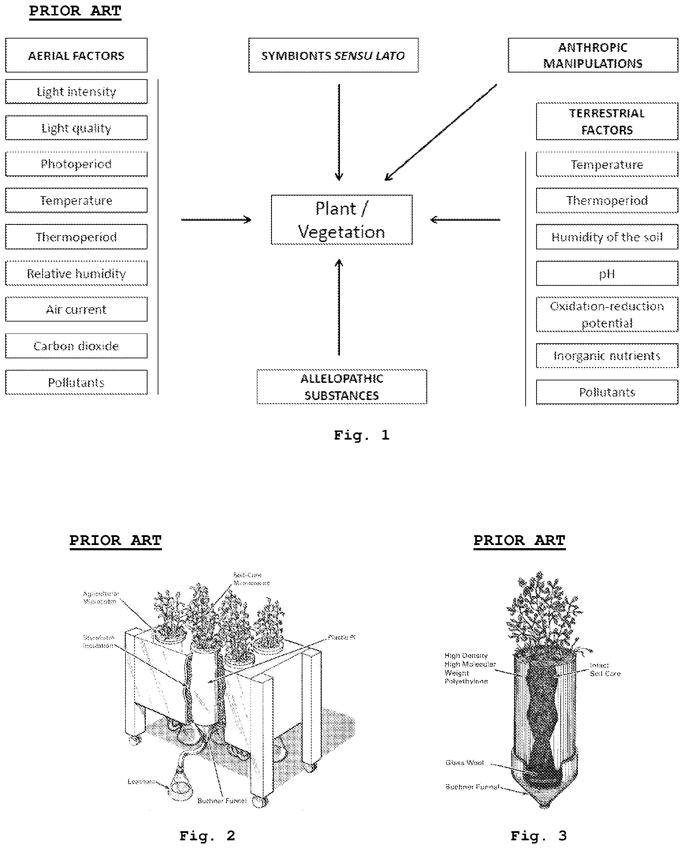

Many environmental factors affect the plant functions and their interaction with plants is extremely articulated and complex. The functionality, growth and development of the radical part of a plant are markedly affected by the characteristics of the terrestrial environment, the best known of which are temperature and thermoperiod, humidity and hydrological characteristics, acidity, oxidation-reduction potential, level of nutrients and their availability. The functionality, growth, and development of the aerial part are markedly affected by the characteristics of the atmospheric environment, the best known of which are intensity and spectral characteristics of the incident light, photoperiod, temperature and thermoperiod, level of relative humidity, carbon-dioxide content in the proximity of photosynthetic organs, speed and directionality of the air currents, duration of leaf moistening. The vegetal functions may be enhanced or depressed also as a result of an articulated network of interactions that the plants have with symbiont organisms, such as viroids, viruses, phytoplasmas, bacteria, fungi, protozoa, parasitic plants, nematodes, phytophagous and sap-sucking arthropods, parasitoid and hyperparasitoid arthropods and higher herbivorous animals, i.e., all the organisms that "live with" plants, interacting therewith in an extremely diversified and complex way, so far still poorly understood. Functionality, growth, and development of the plants can be conditioned also by nutritional imbalances, by allelopathic phenomena and by the presence of pollutants in the environment, including toxic agents of iatrogenic origin. Finally, in the cultivation environment, the vegetal functions may be conditioned also by a series of manipulations carried out deliberately by the farmer that have an impact on the architecture of the plant and on the plant canopy. The environment in which the plants grow is, hence constituted by the complex of all these conditions, both biotic and abiotic, which interact with one another and with the plant system to condition the vital processes the plants (Figure 1).

In the field practice, any modification of the environmental conditions, irrespective of whether it is the consequence of human intervention or derives from spontaneous natural dynamics, may exert beneficial or adverse effects on the plant functions. The effects, either positive or negative, derive from responses at the biochemical level in the single organisms, which respond to the stress through modifications in the primary and secondary metabolism that, in turn, lead to more or less evident phenotypic responses - of biochemical, ultrastructural, cytological, histological, morphological or symptomatological nature - in the plant.

Since the environmental conditions in which the organisms live markedly affect both the metabolic processes of the organisms themselves and the ecophysiological phenomena that the populations the organisms belong to, growing organisms in imposed and controlled environmental conditions remains of fundamental importance for research activities in all branches of physiology and ecophysiology. Management and monitoring the environmental growing conditions become, if possible, even more relevant when the experimental design envisages "multicomponent" systems, based upon the interaction between a number of organisms set in relation with one another by trophic or metabolic relationships, because the interaction between organisms and between organisms and the environment reaches levels of complexity comparable to the ones that occur in natural ecosystems.

By "cosmos" is meant a universe in the sense of a complex and orderly entity; hence, in reference to a plant organism, a "microcosm" will be a reproduction on a small scale of the environment in the plant lives. Consistently, the best a microcosm reproduces the complexity of the environment the plants live in, the most representative it will be of the environment the plants are grown in. In addition, it will be more useful on the experimental level if: a) it will be able to manage and monitor the environmental parameters that act on the metabolism and on the physiology of the vegetal organism, b) it will enable to supply stress factors to plants as it occurs in nature, c) it will enable explanting and samplings in such a way as to minimize the perturbation of the experimental conditions.

In the current state of the art, the equipment that most closely reproduces the characteristics of a microcosm for growing plants is the so-called phytotrone. By the term "phytotrone" (Went F.W., 1950, "The Earhart Plant Research Laboratory", Chronica Botanica 12:91-108; Went F.W. 1957, "The experimental control of plant growth" Waltham, Mass., p. 343), referred to for the first time in 1949 by Frits Warmolt Went, who devised it at the Earhart Plant Research Laboratory, California Institute of Technology of Pasadena, is understood a complex of air-conditioned chambers for experimentation on plants. A phytotrone differs from a greenhouse complex because it enables a more complete control of the plant-growing environment, has apparatuses for control and management of the environmental parameters that enable management of the conditions of growth and detection of the environmental data, and enables simultaneous availability of different environmental conditions and hence comparison of the effects of different environments on vegetal functions. Currently, there is a wide range of commercially available equipment for growing biological material in general and, with particular reference to plants, it is possible to find on the market equipment that comes under the terms "phytotrone", "phytological chamber", "growth chamber", "climatic chamber", "climatic cell" etc., which provides growing conditions for plants that can be widely modulated, with an acceptable precision in the levels of management and control.

All this equipment is, however, far from being versatile for experimental requirements, for example regarding to supply of stress factors to plants, explanting and sampling in conditions of minimal environmental perturbation, continuous analysis of gaseous emissions, application of sensor systems that can be modulated according to type and location, just to mention a few of such requirements. The reason for this lack of versatility is linked to the constructional characteristics of the commercial equipment, that is arranged as a single environment constituted by a fluid-tight chamber. In particular, with reference to plants, which have a radical part and an aerial part that present different *optima* of environmental conditions for their vital functions, the commercial equipment displays the considerable limit of treating the plant as a whole, conditioning the entire plant-growing environment without any possibility of diversifying the environmental conditions between the hypogeal part and the epigeal part, thus resulting in environmental conditions that are very different from the ones that occur in nature. Furthermore, in commercial equipment every operation from outside by the operator - water and nutrient supply, introduction of contaminants, inoculation of pathogens, introduction of parasites, tissue explantation, sampling of air, water and substrate etc. - can be carried out only by accessing directly the whole plant-growing environment, thus causing an inevitable perturbation of the conditions the plants live in.

Furthermore, in the specific case of the microcosm according to what appears in the American Society for Testing and Materials (ASTM), ASTM E 1197-87 (see hereinafter), any sampling in the soil phase imply to dismantle the experimental setup, which would entail the end of the experiment.

The conceptual and structural limits of commercial equipment for growing plants in given conditions become particularly evident when it is necessary to conduct an experiment that presents connotations of particular complexity and in which the environmental conditions are expected to have a considerable effect on the quality and reproducibility of the results. In this case, to overcome the limits typical of the commercial equipment so far available it would be useful a system that: a) will enable a separate management of the environmental parameters regarding the epigeal part and hypogeal part of the plants; b) will enable to work with different plant architectures; c) will enable to supply stress factors in a dosed and controlled way; d) will enable explanting and sampling to be carried out without inducing marked alterations of the current environmental conditions; and e) will enable experimentation of various types of sensors used with variable settings.

The idea of creating a *microcosm for growing plants under biotic and abiotic conditioning* arose from the study of a microcosm dedicated to ecotoxicological studies on the soil system, devised for studying the environmental fate, the ecological effects and the transport into the soil of chemical agents that enter the terrestrial ecosystem, as described by the protocol ASTM E 1197-87 (re-approved in 2004), recently reviewed as ASTM E1197-12. The microcosm dedicated to the terrestrial system referred to in the aforesaid protocol is constituted by cylinders made of plastic material, without side openings and without integrated sensors, that rest on Büchner-type funnels, which enable conveyance of the leachate into containers external to the microcosm, for subsequent analysis. The system of cylinders is housed in a closed compartment in which a thermal conditioning at constant temperature is carried out. No active conditioning is envisaged for the environment of growth of the aerial part of plants eventually grown in the soil contained in the cylinders.

In Figures 2 and 3 are reported some graphic representations of the microcosm according to ASTM E 1197-87.

A search carried out on the website *www.orbit.com* by entering the keyword "microcosm" and the keyword "phytotrone" and selecting the results obtained that have some relevance to the vegetal world has enabled identification of the following documents: CN201662570U_U, GB2160406_A, JP2013165658_A, US20050277169_A1, US20070277744_A1. Of these, only the document CN201662570U_U has revealed some relevance to the microcosm according to the present patent application, as well as a considerable resemblance to the microcosm dedicated to the terrestrial system according to ASTM E 1197-87.

As compared to the microcosm according to ASTM E 1197-87 and to the microcosm disclosed in the document CN201662570U_U, the microcosm according to the present patent application presents various elements in which it differs, such as: a) provision of a epigeal compartment whose environmental parameters can be conditioned; b) integration of the cylinder-type system for growing the radical part of the plant with a box-type growing system, which is more suited to plants displaying a fasciculate radical apparatus; c) the presence of holes in the cylinders and in the boxes for growing the radical part that enable the passage of sampling probes and sensors and d) the presence of an articulated sensor system.

By definition, the *microcosm for growing plants under biotic and abiotic conditioning,* referred to hereinafter more simply by the term "microcosm", is an isolated environment in which it is possible to grow plants in environmental conditions that are imposed and monitored, i.e., controlled in a constant, continuous, and methodical way, said conditions being diversified for the radical part and the aerial part, to supply stress factors of biotic and abiotic type in given doses, and to carry out sampling of tissue, substrate, water present in the soil, soil atmosphere, aerial atmosphere and symbionts inducing only a minimal perturbation of the plant-growing conditions.

The microcosm according to the invention (a prototypal configuration of which is represented in the attached drawings) constitutes an environment in which it is possible to replicate on a laboratory scale what occurs under natural conditions in a plant or in a plant canopy when given environmental conditions are imposed and organisms capable of interacting or interfering with the vegetal functions intervene.

The above microcosm enables tests to be conducted, aimed at:
A. correlating alterations in the environmental conditions with metabolic and phenotypic alterations in order to understand the effects of modifications induced in the environment on the plant functions;
B. developing models of prediction of the responses of the plants to modifications in the environmental conditions;
C. validating models of prediction of the responses of the plants to modifications in the environmental conditions; and
D. developing practical agronomic protocols for inducing useful responses in the plant system by acting on the growth environment.

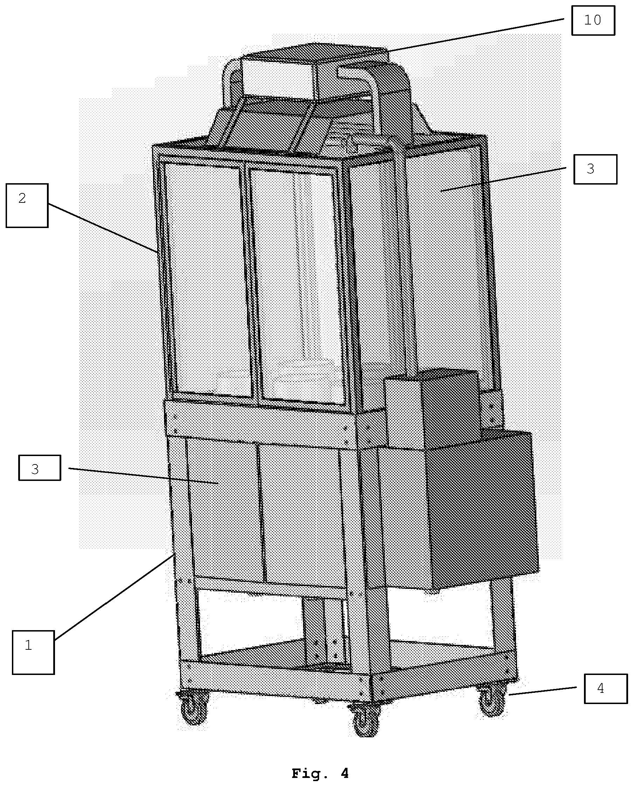

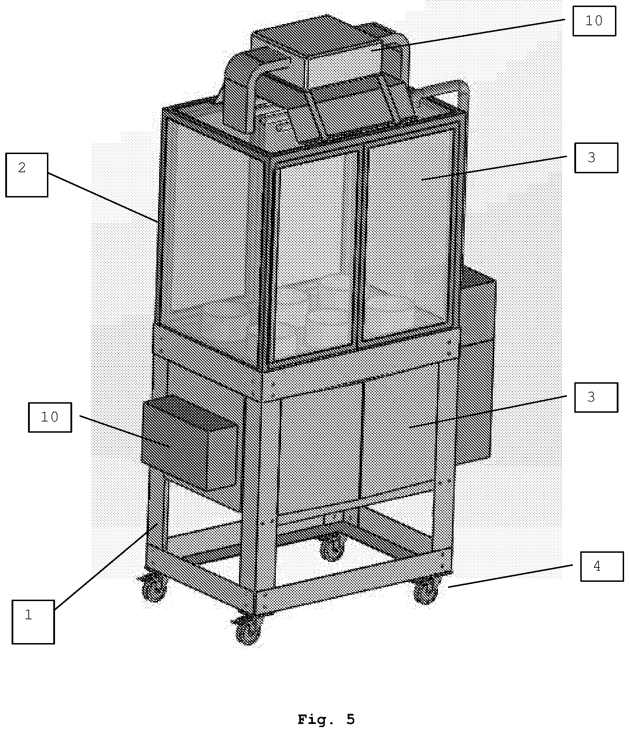

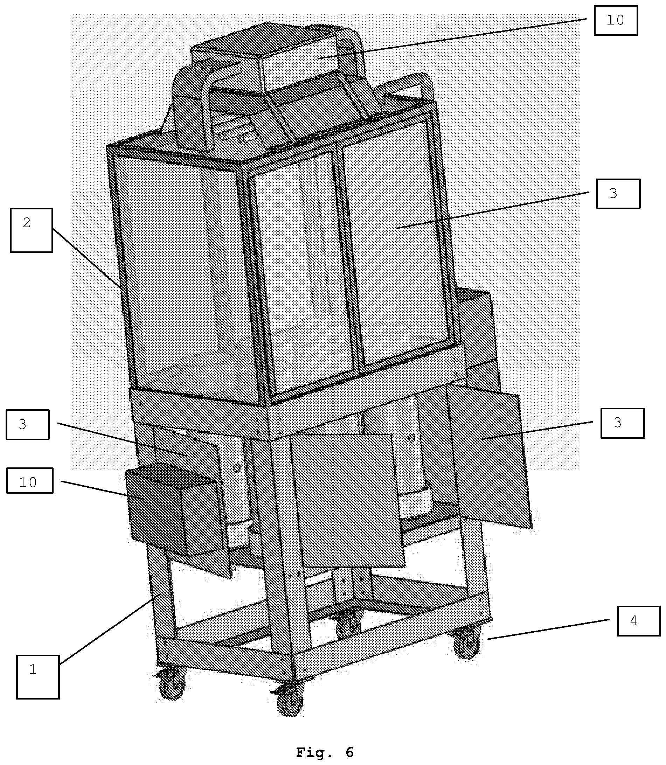

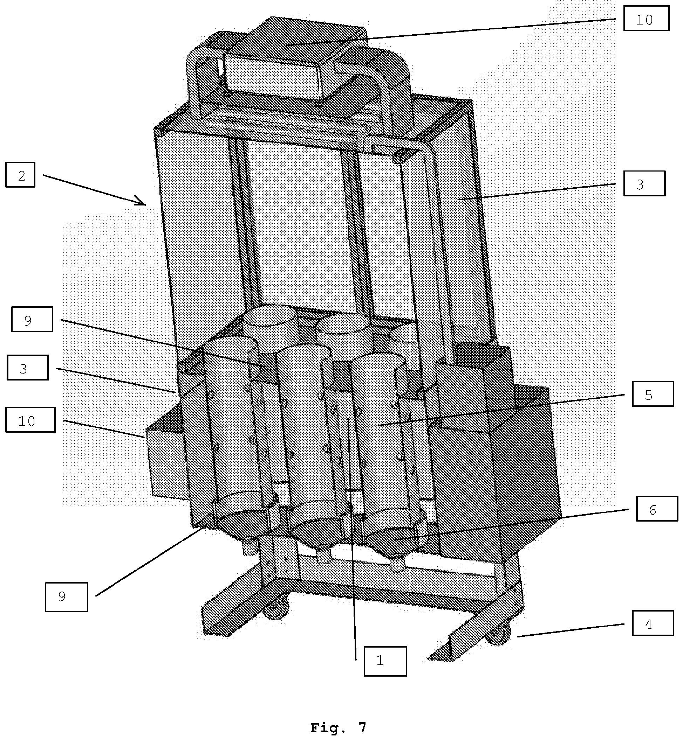

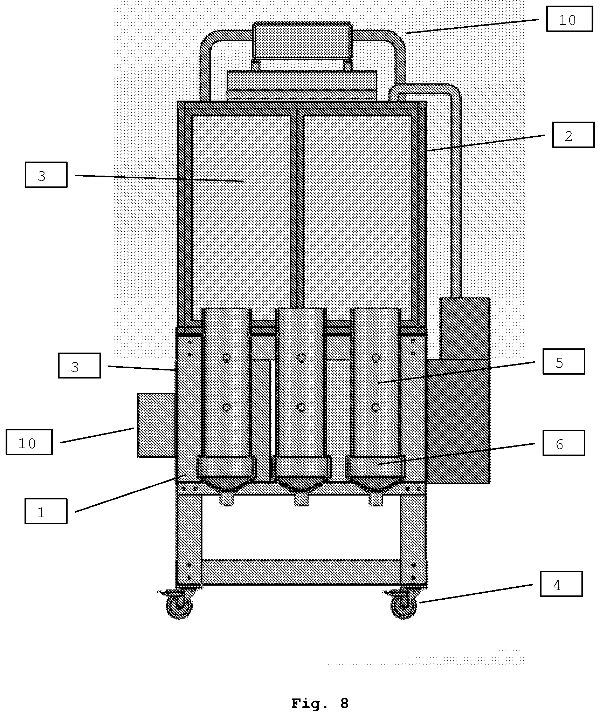

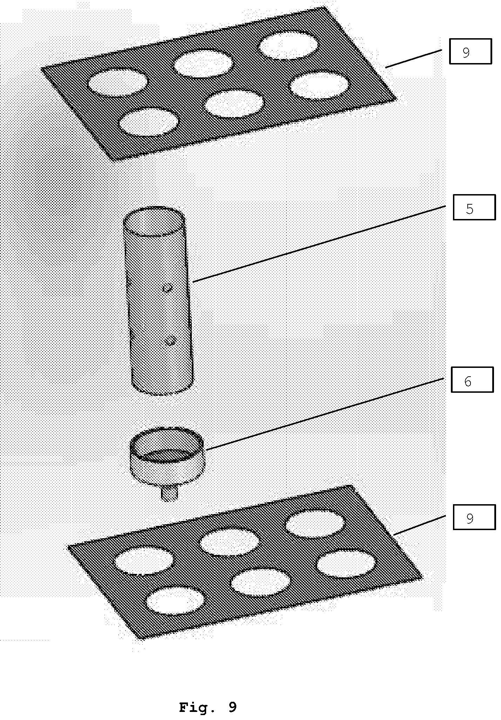

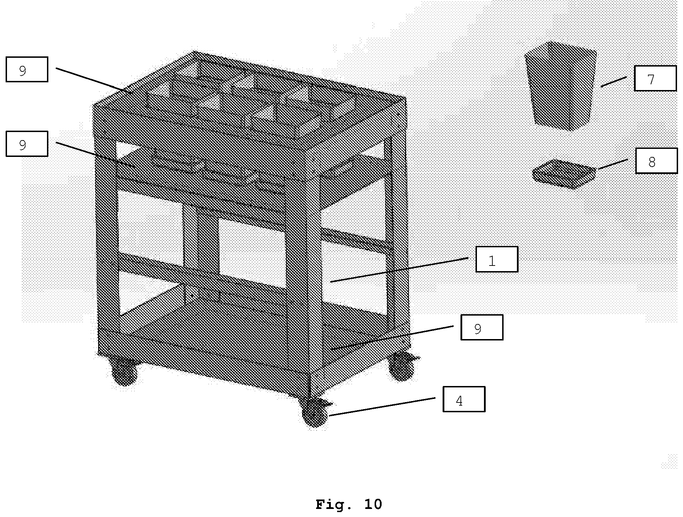

With reference to Figures 4 to 10, according to the present invention the microcosm is a confined environment capable of minimizing the exchanges of energy, molecules, and organisms between the area in which the plants and their symbionts are grown, on one hand, and the outside world, on the other.

According to the invention, the aforesaid confined environment has dimensions such as to guarantee manageability of use, assemblage, and location, without constituting an excessive limit for a significant experimental design.

The microcosm is an apparatus constituted by the complex of two independent chambers: a hypogeal chamber 1 (hereinafter also referred to as "hypogeal compartment"), and an epigeal chamber 2 (hereinafter also referred to as "epigeal compartment").

The apparatus according to the invention envisages a set of components designed to enable active management and constant, continuous and methodical passive control of the microclimatic parameters that affect the life of the plants and their symbionts, as well as enabling constant, continuous and methodical acquisition and processing of the micro-environmental data. The apparatus moreover envisages arrangements useful for housing sensing probes and for carrying out samplings within the epigeal compartment 2 and the hypogeal compartment 1, reducing to a minimum the perturbation of the micro-environmental conditions. The hypogeal chamber 1 and the epigeal chamber 2 are assembled vertically on top of one another so as to constitute a single complex and functionally integrated structure.

The microcosm as a whole is constituted by the following components:
∘ hypogeal chamber 1;
∘ epigeal chamber 2;
∘ system for power supply and general control;
∘ system for monitoring and active managing the thermoperiod in the hypogeal chamber 1;
∘ system for monitoring the humidity of the root substrate in the hypogeal chamber 1;
∘ system for monitoring the pH of the root substrate in the hypogeal chamber 1;
∘ system for monitoring the oxidation-reduction potential of the root substrate in the hypogeal chamber 1;
∘ system for monitoring and active managing the photoperiod in the epigeal chamber 2;
∘ system for monitoring the light intensity in the epigeal chamber 2;
∘ system for monitoring the light spectrum in the epigeal chamber 2;
∘ system for monitoring and active managing the thermoperiod in the epigeal chamber 2;
∘ system for monitoring and active managing the relative humidity in the epigeal chamber 2;
∘ system for monitoring and active managing the air flow in the epigeal chamber 2;
∘ system for monitoring the level of leaf moistening in the epigeal chamber 2;
∘ system for monitoring and active managing the carbon-dioxide content in the epigeal chamber 2; and
∘ system for processing the micro-environmental data detected in a constant, continuous and methodical way in the two chambers: the hypogeal chamber 1 and the epigeal chamber 2.

The hypogeal chamber 1 is the part of the microcosm that is designed for growing the radical part of the plants and of the rhizosphere, i.e., the complex of the roots and the organisms that live in that part of the substrate that is affected by the presence of the roots. In the example described, the hypogeal chamber 1 consists of a panelled compartment, provided with openable panellings 3 to enable internal inspection, insertion of probes, sampling and cleaning. The hypogeal chamber 1 is mobile on wheels 4. The compartment is equipped for housing different types of containers 5, 7 that are to contain the root substrate. All the components of the hypogeal compartment 1, as well as the containers 5, 7 for growing the radical apparatus, are made of a material characterized by durability, resistance to thermal and mechanical stresses and to attack by acids and bases, and low tendency to emit volatile compounds. The aforesaid containers, preferably constituted by cylinders 5 or by boxes 7, may be made of a material that ensures a high degree of transparency so as to enable also visual analysis of the radical growth. The compartment constituting the hypogeal chamber 1 is thermostated through said system for monitoring and actively managing the thermoperiod. Active management of the thermoperiod in the compartment enables, by transmission of heat, a thermoperiod to be obtained also in the substrate contained in the cylinders 5 or in the boxes 7 for growing the radical part of the plants and, hence, in the area of substrate directly explored by the roots of the plants being grown. Probes for detecting the level of humidity, the pH, and the oxidation-reduction potential of the soil are inserted in the root substrate.

The containers 5, 7 for growing the plant roots are interchangeable thanks to the fact that they are housed in guides constituted by interchangeable shelves 9. They have a cylindrical shape 5 or frusto-pyramidal shape 7, and dimensions suited to housing root apparatuses of different size and architecture. The cylindrical containers 5 are the best suited to housing plants with a radical apparatus of a tendentially tap root type, whereas the frusto-pyramidal containers 7 are best suited to housing plants with a radical apparatus of a tendentially fasciculate type. These containers have holes set in adequate positions and whose size enables access to any point of the substrate and of the radical apparatus. The cylinders 5 and the boxes 7 are open-bottomed and are housed the former within funnels 6 with perforated bottom and the latter in saucers 8, thus providing a fluid-tight system that, however, drains off the excess water and is useful also for watering plants from the bottom and enables easy cleaning after dismantling.

The epigeal chamber 2 is the part of the microcosm that is designed for growing the aerial part of the plants and of the phyllosphere, i.e., the complex of the aerial part of the plants and the organisms that live in the volume of atmosphere that is affected by the presence of the aerial part of the plant. The epigeal chamber 2 consists of a panelled compartment, whose structural part which can be applied on the hypogeal chamber 1 and is enclosed by four side panellings 3 and a top panelling 3, where said panellings can be opened and removed to enable internal inspection, insertion of probes, sampling and cleaning. All the components of the epigeal compartment 2 are made of a material characterized by durability, resistance to thermal and mechanical stresses and to the attack of acids and bases and low tendency to emit volatile compounds. The side panellings 3 are moreover made of a material that ensures a high degree of transparency so as to enable also visual analysis of the growth of the aerial part of the plants. These panellings are applied with known systems that enable their replacement when a modification of the type of material used for enclosure is needed. Furthermore, the side panellings 3 can be obscured by simply applying opaque adhesive films on the surface when the absence of exogenous light is needed. The compartment constituting the epigean chamber 2 is provided with a system for monitoring and actively managing the photoperiod, the thermoperiod, the relative humidity and the atmospheric carbon-dioxide content, as well as sensors for monitoring the light intensity, the light spectrum, the rate of the air flow and the level of leaf moistening. Lighting is obtained with lamps capable of emitting photosynthetically active light suited, according to the experimental needs, to stimulating conditions of vegetative growth, reproductive stimulus or both. The lighting system can be modified to envisage the use of monochromatic wavelengths.

Sizing in height of the containers for the roots 5, 6, 7, 8 and, consequently, of the entire hypogeal chamber 1 is designed taking into account the fact that in nature, and in particular in a cultivated field, the radical apparatus of the plants prevalently occupies the part of the soil closest to the surface developing mainly within the first 50-60 cm of depth.

Sizing in width and in depth of the hypogeal chamber 1 is designed taking into account the need to have a minimum number of replicas of the containers for the roots to ensure articulation of the experimental designs and reliability of the results - not less than two replicas per each type of container - and the need for every point of the compartment to be easily accessible by the operator to enable insertion of probes, sampling operations and cleaning of the compartment.

Sizing in height of the epigeal chamber 2 is designed so as to enable plants growing in the microcosm to potentially reach a ratio between the depth of the radical apparatus and the height of the aerial part not less than one, thus limiting as far as possible any dwarfing effect and early ageing of the plants.

Sizing in height of the epigeal chamber 2 also takes into account the need to guarantee the static stability of the microcosm.

Sizing in width and depth of the epigeal chamber 2 is carried out taking into account the need for housing this chamber on top of the hypogeal chamber 1.

According to a peculiar characteristic of the invention, the hypogeal chamber 1 and the epigeal chamber 2 are in communication with one another exclusively through the openings in which the cylinders 5 or the boxes 7 for growing the plants are housed. Hence, when these holes are engaged by the cylinders or by the boxes, the hypogeal chamber 1 and the epigeal chamber 2 are in contact only through the surface of the substrate exposed to the air of the epigeal compartment 2. In this way, the exchanges between the hypogeal compartment 1 and epigeal compartment 2 are advantageously mediated only by the substrate for growing the plants, as it occurs in natural conditions.

It should be pointed out that, according to the experimental needs, the two chambers, the hypogeal chamber 1 and the epigeal chamber 2, according to the present invention, can be used also separately and independently of one another so as to obtain two distinct structures, one constituted by just the epigeal chamber 2, which can be used as a case under which it is possible to grow entire plants in a confined and altogether controlled environment, and the other constituted by just the hypogeal chamber 1, where it is possible to grow entire plants having just the radical part in a confined and controlled environment. The entire microcosm can be used also without the aforementioned systems for detection and active management of the micro-environmental parameters, also activating only some of them, in case for experimental requirements it is necessary to have no control or only partial control of the plant-growing environment. The same refers to the individual chambers used separately.

As has already been specified, the microcosm according to the present invention is equipped with a system for power supply and general control, a system for monitoring and actively managing the thermoperiod in the hypogeal chamber 1, a system for monitoring of the humidity of the root substrate in the hypogeal chamber 1, a system for monitoring the pH of the root substrate in the hypogeal chamber 1, a system for monitoring of oxidation-reduction potential of the root substrate in the hypogeal chamber 1, a system for monitoring and actively managing the photoperiod in the epigeal chamber 2, a system for monitoring the light intensity in the epigeal chamber 2, a system for monitoring the light spectrum in the epigeal chamber 2, a system for monitoring and actively managing the thermoperiod in the epigeal chamber 2, a system for monitoring and actively managing the relative humidity in the epigeal chamber 2, a system for monitoring and actively managing the air flow in the epigeal chamber 2, a system for monitoring the level of leaf moistening in the epigeal chamber 2, a system for monitoring and actively managing the carbon-dioxide content in the epigeal chamber 2, and a system for processing the micro-environmental data detected in a constant, continuous and methodical way in the two different chambers 1, 2.

A second peculiar characteristic of the invention consists in the fact that a system for thermal conditioning is provided based upon the use of Peltier cells 10 in so far as it is light, far from cumbersome and capable of managing both heating and cooling, as well as ventilation.

The data-transmission system may be either wired or wireless, the latter being useful for managing the microcosm also remotely and also in locations outside the laboratory, especially locations that are not readily accessible.

In a preferred embodiment of the invention, the hypogeal chamber 1 is obtained by assembling the following components:
∘ a load-bearing structure constituted by L-shaped sectional elements made of 304 stainless steel having a thickness of approximately 3 mm, with sides of approximately 100 cm x 70 cm, and a height of approximately 100 cm, with cultivation surface set at 115 cm from the treading surface, bolted with stainless steel bolts and mobile on four swivel wheels made of stainless steel having a diameter of approximately 100 mm;
∘ side panellings made of thermally insulated aluminium, a fixed one on one of the minor sides, and three with hinged leaves, which can be opened by means of handles, on the other three sides;
∘ a removable and interchangeable top shelf, made of 304 stainless steel having a thickness of approximately 3 mm with holes designed to house six cylinders that each have a diameter of approximately 20 cm;
∘ a removable and interchangeable top shelf, made of 304 stainless steel having a thickness of approximately 3 mm with holes designed to house four cylinders that each have a diameter of approximately 20 cm;
∘ a removable and interchangeable top shelf, made of 304 stainless steel having a thickness of approximately 3 mm with holes designed to house two cylinders that each have a diameter of approximately 20 cm;
∘ a removable and interchangeable top shelf, made of 304 stainless steel having a thickness of approximately 3 mm, with openings designed to house six quadrangular boxes having a frusto-pyramidal shape, each with a major base of approximately 23 cm x 18 cm;
∘ a removable and interchangeable top shelf, made of 304 stainless steel having a thickness of approximately 3 mm, with openings designed to house two quadrangular boxes having a frusto-pyramidal shape, each with a major base of approximately 33 cm x 25 cm;
∘ a removable and interchangeable bottom shelf, made of 304 stainless steel having a thickness of approximately 3 mm, with holes designed to house six funnels of a Büchner type made of porcelain, each having a saucer diameter of not less than 20 cm;
∘ a removable and interchangeable bottom shelf, made of 304 stainless steel having a thickness of approximately 3 mm, with holes designed to house four funnels of a Büchner type made of porcelain, each having a saucer diameter of not less than 20 cm;
∘ a removable and interchangeable bottom shelf, made of 304 stainless steel having a thickness of approximately 3 mm, with holes designed to house two funnels of a Büchner type made of porcelain, each having a saucer diameter of not less than 20 cm;
∘ a removable and interchangeable bottom shelf, made of 304 stainless steel of thickness 3 mm and bent back on the four sides for a length of at least 10 cm, of dimensions of approximately 100 cm x 70 cm;
∘ cylinders made of polycarbonate having a diameter of approximately 20 cm and a height of approximately 60 cm provided with three series of four openings having a diameter of approximately 30 mm, arranged along the circumference at a distance of 90° with respect to one another, and set at 15, 30, and 45 cm from the top edge of the cylinder;
∘ funnels of a Büchner type made of porcelain, having a saucer diameter of not less than 20 cm and a height contained within 20 cm;
∘ bottomless quadrangular boxes having a frusto-pyramidal shape with a major base of approximately 23 cm x 18 cm and a height of approximately 30 cm, provided with two openings having a diameter of approximately 30 mm that are arranged on each of the long side and are set at approximately 15 cm from the top edge of the box;
∘ bottomless quadrangular boxes having a frusto-pyramidal shape, with a major base of approximately 33 cm x 25 cm and a height of approximately 30 cm, provided with two openings having a diameter of approximately 30 mm that are arranged on each of the short side and are set at approximately 15 cm from the top edge of the box;
∘ saucers made of stainless steel for quadrangular boxes having a frusto-pyramidal shape, with a major base of approximately 23 cm x 18 cm; and
∘ saucers made of stainless steel for quadrangular boxes having a frusto-pyramidal shape, with a major base of approximately 33 cm x 25 cm;

Accordingly, the epigeal chamber 2 is obtained by assembling the following components:
∘ a load-bearing structure made up of bolted aluminium sectional elements having a length and width of approximately 100 cm x 70 cm and a height of approximately 94 cm; and
∘ polycarbonate panellings, those on the major side being hinged and divided into two leaves of equal dimensions that can be opened by means of handles, the ones on the minor side and the one on the top side being bolted to the load-bearing structure.

In the example of embodiment that has been described, the system for power supply and general control is implemented with a low-voltage Gemini switchboard manufactured by ABB, with IP66 degree of protection, supplied by just one cable connected to a current outlet from the 220-V mains supply. Distribution of the supply to the loads starts from a 40-A main (magnetothermal/differential) circuit breaker, for protection of a subset of magnetothermal switches (6-A, 10-A, and 16-A switches) that enable supply of the various service loads.

The system for managing the thermoperiod in the hypogeal chamber 1 is obtained using a 250-W, 24-V Peltier plate, type AA-250-24-44-00, manufactured by Laird Technologies, a 220/24-V, 15-A power supply, of the Tracopower TXL350-24S type, a programmer controller, model PR-59, manufactured by Laird Technologies (provided with a software program for management and control of the power of the plates and speed of the fans) and a temperature probe of an NTC type. The Peltier module is mounted on a side panel of the hypogeal chamber 1, with the heat-exchange fan set directly inside the chamber with the other side communicating with the external environment. Regulation of the temperature is obtained by recirculation of air, which, pushed by a fan, traverses the heat exchanger of the plate (for heating or cooling, according to the function set by the regulator) until the temperature set point is reached. The regulation chain is the following: the controller PR-59, by means of the NTC temperature sensor located within the chamber, acquires the value of the temperature in the chamber and, after it has set it in relation with the temperature set point, carries out a regulation of the current that the power supply has to deliver to the Peltier device. According to the intensity and direction of the current, the Peltier device heats or cools the primary heat-exchange plate, with consequent regulation of the temperature of the air pushed into the chamber by the fan connected thereto. In addition to setting of the temperature, on the control panel of the controller it is possible to regulate supply of the fan on the hot side and on the cold side of the Peltier plate, and, consequently the speed of the air introduced. The controller on the plate may be set so that it carries out a regulation of a proportional type, or else a PD type, or else a PID type. The controller is connected to the PC by means of an RS-232 serial port.

Accordingly, the system for managing the thermoperiod in the epigeal chamber 2 is obtained by means of a 250-W, 24-V Peltier plate, type AA-250-24-44-00, manufactured by Laird Technologies, a 220/24-V, 15-A power supply, of the Tracopower TXL350-24S type, a programmer controller, model PR-59, manufactured by Laird Technologies (provided with a software program for management and control of the power of the plates and of the speed of the fans), and a temperature probe of an NTC type. The Peltier module is housed outside the compartment and thermoregulation is obtained by exchange of air taken in from the external environment and introduced into the chamber by means of a duct made of PVC plastic material. The regulation chain is the same as the one described for the hypogeal chamber 1.

The choice of the Peltier cells used for control of the thermoperiod in the two chambers is the fruit of an accurate study during the step of design of the microcosm. An alternative to the Peltier cells would have conditioned the choice of pre-set strategic solutions in view of the final aim. Lightness, ease of mechanical coupling and decoupling between the hypogeal and the epigeal parts and the specificity of contemplating a unique cooling, heating and ventilation apparatus have driven the choice towards the use of Peltier cells for thermoregulation of the two chambers.

The system for managing the photoperiod is implemented with 55-W neon lamps of the Sylvania Linx-I type, which emit photosynthetically active light suited to support both the stage of growth and the stage of flowering of the plants. The lamps are controlled by ON/OFF timers, programmable right round the clock so as to generate a pre-set photoperiod according to the experimental requirements. A sensor for monitoring and recording the intensity of the light emitted in a constant, continuous and methodical way is placed in the chamber.

The system for managing the relative humidity is obtained with a humidifier of an ultrasonic type manufactured by Cornwall Electronics, with a 220-V power supply and with a 6-litre tank fixed up against the structure. A delivery duct, provided with 40-mm tubes and accessories made of PVC, transfers the water vapour generated into the compartment of the epigeal chamber. A humidity sensor set within the chamber regulates the humidity according to the set point by ON/OFF switching of the humidifier itself.

In accordance with what has been described so far, it may be said that the chief feature of novelty of the microcosm 1 according to the present invention, which marks it off from the equipment commonly used for growing plants under controlled conditions, lies in the fact that it has available two different chambers that enable growing the aerial part and the radical part of the plants under different environmental conditions in order to reproduce as closely as possible what occurs in nature.

It should be noted, for instance, that under natural conditions, given the low mobility of gases in the soil and given that soil and atmosphere communicate only through the surface of the soil, gas molecules emitted by roots and aerial plant parts have little likelihood of coming into contact with one another and with the whole plant. In common laboratory equipment for growing plants under controlled conditions, since the plant as a whole is grown in a unique compartment, the volatile substances emitted by the roots and the leaves spread freely in the atmosphere of the single growing compartment, thus potentially creating conditions of non-natural interaction between these two parts mediated by the volatile substances, for example, through the surfaces of the draining holes or through the lateral surfaces when the sod is raised, during insertion of the probes or during sampling involving the soil compartment. According to the invention described, instead, the atmosphere of the hypogeal compartment 1 and that of the epigeal compartment 2 are separated, and the two chambers, once the cylinders 5 or the boxes 7 have been set on the funnels 6 or on the saucers 8, respectively, and have been filled with the substrate, can communicate only through the substrate itself, which has a free surface towards the atmosphere of the hypogeal compartment 1 through the holes provided in the boxes 7 and in the cylinders 5 and a free surface towards the atmosphere of the epigeal compartment 2 through the part of the substrate that faces the aerial part. In this way, the microcosm mimics quite faithfully what occurs under natural conditions.

In addition, it should be noted that in the laboratory equipment commonly used for growing plants under controlled conditions, sampling of air in the soil and in the atmospheric as well as picking samples from the root substrate, from the roots and from the aerial parts of the plant necessarily occurs in the atmosphere of the single compartment. Instead, in the device forming the subject of the present invention, the physical separation between the hypogeal compartment 1 and the epigeal compartment 2 enables sampling the air in the soil and in the atmosphere and sampling the substrate and the different plant parts, independently in the environmental conditions of the particular portion and without perturbing the environmental conditions of the portion not involved in the sampling.

Finally, it is to be noted that in the laboratory equipment commonly used for growing plants under controlled conditions, sampling the roots and the root substrate is performed through digging the substrate, even after extraction of the soil sod containing the roots from its container. Both these operations may damage the radical apparatus and may markedly perturb the soil environment. Advantageously, according to the present invention, the presence of holes of adequate size in the cylinders and in the boxes used for growing the plants enables sampling of the air present in the soil and sampling of the substrate and radical explants with practically no perturbation of the integrity of the radical apparatus and of the environmental conditions the plants live in.

The microcosm is useful to support research activities in many different fields, for example in chemical ecology, plant physiology, plant ecophysiology, agroecology, plant pathology, plant parasitology, environmental microbiology, soil chemistry and agronomy.

For instance, the microcosm may find direct application in experimental setups aimed at studying:
∘ emission of volatile organic compounds from the soil and/or plant system;
∘ interaction between plant and environmental factors;
∘ interaction between plants and parasites;
∘ interaction between plants and pathogens;
∘ interaction between pathogens and parasites that affect the plant system;
∘ interaction between plants and agrochemicals;
∘ interaction between agrochemicals and soil;
∘ interaction between contaminants and soil; and
∘ interaction between contaminants and soil system + vegetation.

The present invention has been described and illustrated according to a preferred embodiment, but it is evident that any person skilled in the branch may make modifications and/or substitutions that are functionally or technically equivalent, without thereby departing from the sphere of protection as specified in the annexed claims.

### LEGEND:

- 1:: hypogeal chamber
- 2:: epigeal chamber
- 3:: panelling of the hypogeal chamber and of the epigeal chamber
- 4:: wheel
- 5:: cylinder
- 6:: funnel
- 7:: frusto-pyramidal box
- 8:: saucer
- 9:: shelf
- 10:: Peltier cell of the epigeal chamber and of the hypogeal chamber

## Claims

1. A microcosm for growing plants under biotic and abiotic conditioning, to obtain an environment in which to replicate on a laboratory scale what occurs under natural conditions in a plant or in a plant canopy when given environmental conditions are imposed and organisms intervene that are capable of interacting or interfering with the plant functions, said microcosm being constituted by an apparatus that comprises in combination:
two chambers independent of one another, of which one is a hypogeal chamber (1), which is the part of the apparatus or microcosm that is designed for growing the radical part of the plants and the rhizosphere, and the other is an epigeal chamber (2), which is the part of the apparatus or microcosm that is designed for growing the aerial part of the vegetal systems and the phyllosphere, and further comprises:
- means for active management of the microclimatic parameters that affect the life of the plants and their symbionts;
- means for passive monitoring of the microclimatic parameters that affect the life of the plants and their symbionts;
- means for acquisition and processing in a constant, continuous and methodical way of the data regarding the micro-environmental parameters; and
- means for housing monitoring probes and for carrying out samplings within the epigeal compartment (2) and the hypogeal compartment (1), reducing to a minimum the perturbation of the micro-environmental conditions;
wherein the hypogeal chamber (1) and the epigeal chamber (2) are assembled vertically on top of one another so as to constitute a single complex and
functionally integrated structure,
said microcosm being **characterized in that** said means for active management, passive monitoring, acquisition and processing in a constant, continuous and methodical way of the environmental parameters comprise:
- a system for power supply and general control;
- a system for monitoring and actively managing the thermoperiod in the hypogeal chamber (1);
- a system for monitoring the humidity of the root substrate in the hypogeal chamber (1);
- a system for monitoring the pH of the root substrate in the hypogeal chamber (1);
- a system for monitoring the oxidation-reduction potential of the root substrate in the hypogeal chamber (1) ;
- a system for monitoring and actively managing the photoperiod in the epigeal chamber (2);
- a system for monitoring the light intensity in the epigeal chamber (2);
- a system for monitoring the light spectrum in the epigeal chamber (2);
- a system for monitoring and actively managing the thermoperiod in the epigeal chamber (2);
- a system for monitoring and actively managing the relative humidity in the epigeal chamber (2);
- a system for monitoring and actively managing the air flow in the epigeal chamber (2);
- a system for monitoring the level of leaf moistening in the epigeal chamber (2);
- a system for monitoring and actively managing the carbon-dioxide content in the epigeal chamber (2); and
- a system for processing the micro-environmental data detected in a constant, continuous and methodical way in the two chambers: the hypogeal chamber (1) and the epigeal chamber (2).

2. The microcosm according to the preceding claim **characterized in that** said hypogeal chamber (1) consists of a panelled compartment, provided with openable panellings (3) to enable internal inspection, insertion of probes, samplings, explanting and cleaning.

3. The microcosm according to the preceding claim **characterized in that** said compartment of the hypogeal chamber (1) is equipped for housing different types of containers (5, 6, 7, 8) that are to contain the root substrate suited to different architectures of the radical apparatus.

4. The microcosm according to the preceding claim **characterized in that** said containers are constituted by cylinders (5) and/or boxes (7) and corresponding saucers (8) and may be made of a material that ensures a high degree of transparency so as to enable also visual analysis of the root growth.

5. The microcosm according to Claim 2 **characterized in that** the compartment constituting the hypogeal chamber (1) is thermostated through said system for monitoring and actively managing the thermoperiod.

6. The microcosm according to Claim 3 **characterized in that** probes for monitoring the level of humidity, the pH and the oxidation-reduction potential of the soil are inserted in the root substrate.

7. The microcosm according to Claim 3 **characterized in that** the containers (5, 6, 7, 8) for growing the roots are interchangeable thanks to the fact that they are housed in guides constituted by interchangeable shelves (9).

8. The microcosm according to Claim 3 **characterized in that** said containers (5, 7) have holes in adequate positions and of dimensions such as to enable access to each point of the substrate and of the roots.

9. The microcosm according to Claim 1 **characterized in that** the epigeal chamber (2) consists of a panelled compartment, the structural part of which can be applied on the hypogeal chamber (1) and is enclosed by four side panellings (3) and a top panelling (3), wherein said panellings can be opened and removed to enable internal inspection, insertion of probes, samplings, explanting and cleaning.

10. The microcosm according to the preceding claim, **characterized in that** the side panellings (3) of the epigeal chamber (2) and of the hypogeal chamber (1) are replaceable and may be made of a material that ensures a high degree of transparency so as to enable also visual analysis of the growth of the aerial part and of the radical part.

11. The microcosm according to the preceding claim **characterized in that**, when it is necessary to carry out tests that impose the absence of exogenous light, said side panellings (3) can be obscured by simply applying opaque adhesive films on the surface.

12. The microcosm according to Claim 10 or Claim 11 **characterized in that** the compartment constituting the epigeal chamber (2) is provided with a system for monitoring and actively managing the photoperiod, the thermoperiod, the relative humidity and the atmospheric carbon-dioxide content, as well as with sensors for monitoring of light intensity, light spectrum, rate of air flow, and level of leaf moistening.

13. The microcosm according to the preceding claim **characterized in that** the system for monitoring and actively managing the photoperiod envisages a lighting obtained with lamps capable of emitting photosynthetically active light suited, according to the experimental needs, to stimulating conditions of vegetative growth, reproductive stimulus or both.

14. The microcosm according to Claim 3 **characterized in that** the sizing in height of the containers (5, 7) for the radical part of the plants and, consequently, of the entire hypogeal chamber (1) is designed taking into account the fact that in nature, and in particular in a cultivated field, the radical part of the plants develops prevalently in the uppermost part of the soil and mainly in the first 50-60 cm of depth.

15. The microcosm according to the preceding claim **characterized in that** the sizing in width and in depth of the hypogeal chamber (1) is made taking into account the need to have a minimum number of replicas of the root containers to ensure articulation of the experimental design and reliability of the results - not less than two replicas per each type of container - and to face the need for each point of the compartment to be easily reached by the operator for probe insertion, samplings and cleaning of the compartment.

16. The microcosm according to Claim 9 **characterized in that** the sizing in height of the epigeal chamber (2) is designed so as to enable plants growing in the microcosm to potentially reach a ratio between the depth of the radical apparatus and the height of the aerial part not less than one, thus limiting as far as possible any dwarfing effect and early ageing of the plants.

17. The microcosm according to Claim 3 **characterized in that** the hypogeal chamber (1) and the epigeal chamber (2) are in communication with one another exclusively through the openings in which the root containers (5, 6, 7, 8) are housed, hence, when these holes are engaged by the root containers, the hypogeal chamber (1) and the epigeal chamber (2) are in contact only through the surface of the substrate exposed to the air of the epigeal compartment (2), thus obtaining that the exchanges between the hypogeal compartment (1) and epigeal compartment (2) are advantageously mediated only by the substrate for growing the plants, as it occurs in natural conditions.

18. The microcosm according to Claim 1 **characterized in that** said systems for monitoring and actively managing the thermoperiod of the hypogeal chamber (1) and of the epigeal chamber (2) envisage use of Peltier cells (10), which are light, far from cumbersome, capable of managing both the step of heating and the step of cooling, and capable of managing also the ventilation step.

19. The microcosm according to any one of the preceding claims **characterized in that** said hypogeal chamber (1) is mounted on wheels (4).

## Patentansprüche

1. Mikrokosmos zum Wachsen von Pflanzen unter biotischen und abiotischen Bedingungen, um eine Umgebung zu erhalten, in der man im Labormaßstab replizieren kann, was unter natürlichen Bedingungen in einer Pflanze oder unter einem Pflanzendach auftritt, wenn gegebene Umweltbedingungen auferlegt werden und Organismen eingreifen, die in der Lage sind, mit den Pflanzenfunktionen in Wechselwirkung zu treten oder mit ihnen zu interferieren, wobei der Mikrokosmos durch eine Vorrichtung gebildet wird, die, in Kombination, folgendes umfasst:
zwei Kammern, die unabhängig voneinander sind, von denen die eine eine hypogeale Kammer (1) ist, die Teil der Vorrichtung oder des Mikrokosmos ist, die zum Wachsen des Wurzelanteils der Pflanzen und der Rhizosphäre ausgelegt ist, und der andere Teil eine epigeale Kammer (2) ist, die Teil der Vorrichtung oder des Mikrokosmos ist, die ausgelegt ist zum Wachsen des der Luft ausgesetzten Teils des vegetalen Systems und der Phyllospäre, und die weiterhin umfasst:
- Mittel zum aktiven Management der mikroklimatischen Parameter, die das Leben der Pflanzen und ihrer Symbionten beeinflussen;
- Mittel zum passiven Beobachten der mikroklimatischen Parameter, die das Leben der Pflanzen und ihrer Symbionten beeinflussen;
- Mittel zur Erlangung und Verarbeitung in einer konstanten, kontinuierlichen und methodischen Weise der Daten, die sich auf die Mikroumweltparameter beziehen; und
- Mittel zur Unterbringung von Beobachtungssonden und zur Durchführung von Probenentnahmen innerhalb des epigealen Kompartiments (2) und des hypogealen Kompartiments (1), wodurch die Störung der Mikroumweltbedingungen auf ein Minimum verringert wird;
wobei die hypogeale Kammer (1) und die epigeale Kammer (2) vertikal aufeinander angeordnet sind, sodass sie einen einzelnen Komplex und eine funktional eingebaute Struktur bilden,
wobei der Mikrokosmos **dadurch gekennzeichnet ist, dass** die Mittel zum aktiven Management, zum passiven Beobachten, zur Gewinnung und zur Verarbeitung in einer konstanten, kontinuierlichen und methodischen Weise der Umweltparameter folgendes umfassen:
- ein System zur Energieversorgung und zur allgemeinen Kontrolle;
- ein System zum Beobachten und zum aktiven Management der Wärmedauer in der hypogealen Kammer (1);
- ein System zum Beobachten der Feuchtigkeit des Wurzelsubstrats in der hypogealen Kammer (1);
- ein System zum Beobachten des pH-Werts des Wurzelsubstrats in der hypogealen Kammer (1);
- ein System zum Beobachten des Oxidations-Reduktions-Potentials des Wurzelsubstrats in der hypogealen Kammer (1);
- ein System zum Beobachten und aktiven Verwalten der Fotoperiode in der epigealen Kammer (2);
- ein System zum Beobachten der Lichtintensität in der egigealen Kammer (2);
- ein System zum Beobachten des Lichtspektrums in der epigealen Kammer (2);
- ein System zum Beobachten und aktiven Verwalten der relativen Feuchtigkeit in der epigealen Kammer (2);
- ein System zum Beobachten und aktiven Verwalten des Luftstroms in der epigealen Kammer (2);
- ein System zum Beobachten des Grades der Blattbefeuchtung in der epigealen Kammer (2);
- ein System zum Beobachten und aktiven Verwalten des Kohlendioxidgehalts in der epigealen Kammer (2); und
- ein System zur Verarbeitung der Mikroumweltdaten, die in einer konstanten, kontinuierlichen und methodischen Weise in den zwei Kammern: der hypogealen Kammer (1) und der epigealen Kammer (2) gewonnen wurden.

2. Mikrokosmos nach dem vorhergehenden Anspruch, **dadurch gekennzeichnet, dass** die hypogeale Kammer (1) aus einem mit einer Verkleidung versehenen Kompartiment besteht, welches mit öffnungsfähigen Verkleidungen (3) versehen ist, um eine interne Inspektion, Zufuhr von Sonden, Proben, Herausnehmen und Reinigen zu ermöglichen.

3. Mikrokosmos nach dem vorhergehenden Anspruch, **dadurch gekennzeichnet, dass** das Kompartiment der hypogealen Kammer (1) ausgestattet ist, um verschiedene Arten von Behältern (5, 6, 7, 8) aufzunehmen, welche die Wurzelsubstrate enthalten sollen, welche geeignet sind, um verschiedene Architekturen der Wurzelvorrichtung zu enthalten.

4. Mikrokosmos nach dem vorhergehenden Anspruch,
**dadurch gekennzeichnet, dass** die Behälter durch Zylinder (5) und/oder durch Schachteln (7) und entsprechende Untersetzer (8) gebildet werden, und die aus einem Material herstellbar sind, welches einen hohen Grad an Transparenz sicherstellt, um auch eine visuelle Analyse des Wurzelwachstums zu ermöglichen.

5. Mikrokosmos nach Anspruch 2,
**dadurch gekennzeichnet, dass** das die hypogeale Kammer (1) bildende Kompartiment durch das genannte System temperaturgeregelt ist, um die Wärmedauer zu beobachten und aktiv zu managen.

6. Mikrokosmos nach Anspruch 3,
**dadurch gekennzeichnet, dass** die Sonden zum Beobachten des Feuchtigkeitsniveaus, des pH-Wertes und des Oxidations-Reduktions-Potentials des Bodens im Wurzelsubstrat eingesetzt sind.

7. Mikrokosmos nach Anspruch 3,
**dadurch gekennzeichnet, dass** die Behälter (5, 6, 7, 8) zum Wachstum der Wurzeln aufgrund der Tatsache, dass sie in Führungen eingebaut sind, welche durch auswechselbare Regalböden (9) gebildet werden, austauschbar sind.

8. Mikrokosmos nach Anspruch 3,
**dadurch gekennzeichnet, dass** die Behälter (5, 7) an adäquaten Positionen Löcher haben und diese Abmessungen aufweisen, sodass sie in der Lage sind, jeden Punkt des Substrats und der Wurzeln zugänglich zu machen.

9. Mikrokosmos nach Anspruch 1,
**dadurch gekennzeichnet, dass** die epigeale Kammer (2) aus einem verkleideten Kompartiment besteht, wobei der strukturelle Teil hiervon auf die hypogeale Kammer (1) aufgebracht werden kann und durch vier Seitenverkleidungen (3) und eine Verkleidung am oberen Ende (3) eingeschlossen ist, wobei die Verkleidungen geöffnet werden können und entfernbar sind, um eine interne Inspektion, die Insertion von Sonden, Stichproben, das Herausnehmen und das Reinigen zu ermöglichen.

10. Mikrokosmos nach dem vorhergehenden Anspruch,
**dadurch gekennzeichnet, dass** die Seitenverkleidungen (3) der epigealen Kammer (2) und der hypogealen Kammer (1) austauschbar sind und aus einem Material bestehen können, welches einen hohen Grad an Transparenz gewährleistet, um auch eine visuelle Analyse des Wachstums des an der Luft befindlichen Teils und des Wurzelteils zu ermöglichen.

11. Mikrokosmos nach dem vorhergehenden Anspruch,
**dadurch gekennzeichnet, dass**, wenn es notwendig ist, Prüfungen durchzuführen, die die Abwesenheit von exogenem Licht notwendig machen, die Seitenverkleidungen (3) durch einfaches Anbringen von opaken Haftfilmen auf der Oberfläche abgedeckt werden können.

12. Mikrokosmos nach Anspruch 10 oder Anspruch 11,
**dadurch gekennzeichnet, dass** das Kompartiment, welches die epigeale Kammer (2) bildet, mit einem System zum Beobachten und aktiven Verwalten der Photodauer, der Thermodauer, der relativen Feuchtigkeit und des atmosphärischen Kohlendioxidgehalts ausgestattet ist, als auch mit Sensoren zum Beobachten der Lichtintensität, des Lichtspektrums, der Luftbewegung und des Grades der Blattbefeuchtung.

13. Mikrokosmos nach dem vorgehenden Anspruch,
**dadurch gekennzeichnet, dass** das System zum Beobachten und zum aktiven Managen der Photodauer eine Beleuchtung vorsieht, die durch Lampen erhalten wird, welche zum Imitieren von photosynthetisch aktivem Licht befähigt sind, entsprechend den experimentellen Notwendigkeiten, um Bedingungen zum vegetativen Wachstum, einen reproduktiven Stimulus oder beides anzuregen.

14. Mikrokosmos nach Anspruch 3,
**dadurch gekennzeichnet, dass** die Abmessungen bzgl. der Höhe der Behälter (5, 7) für den Wurzelteil der Pflanzen, und, dementsprechend, der gesamten hypogealen Kammer (1) so ausgelegt sind, dass sie die Tatsache berücksichtigen, dass in der Natur, und insbesondere in einem kultivierten Feld, der Wurzelanteil der Pflanzen sich vorherrschend im obersten Teil des Bodens entwickelt und hauptsächlich in den ersten 50 bis 60 cm Tiefe.

15. Mikrokosmos nach dem vorhergehenden Anspruch,
**dadurch gekennzeichnet, dass** die Abmessungen in der Breite und in der Tiefe der hypogealen Kammer (1) so ausgelegt sind, dass sie die Notwendigkeit berücksichtigen, eine minimale Anzahl an Replikas der Wurzelbehälter vorliegen zu haben, um ein Zusammenspiel des experimentellen Designs sicherzustellen und eine Verlässlichkeit der Ergebnisse - nicht weniger als zwei Replikas für jede Art von Behältern -, um der Tatsache gerecht zu werden, dass jeder Punkt des Kompartiments durch die Bedienungsperson zur Sondeninsertion, zur Probennahme und zur Reinigung des Kompartiments einfach erreichbar ist.

16. Mikrokosmos nach Anspruch 9,
**dadurch gekennzeichnet, dass** die Abmessungen in der Höhe der epigealen Kammer (2) so ausgelegt sind, dass sie das Wachsen von Pflanzen in dem Mikrokosmos ermöglichen, um potentiell ein Verhältnis zwischen der Tiefe der Wurzelvorrichtung und der Höhe des an der Luft befindlichen Teils zu ermöglichen, das nicht weniger ist als eins, sodass soweit wie möglich jeder Verzwergungseffekt und jede frühzeitige Alterung der Pflanzen vermieden wird.

17. Mikrokosmos nach Anspruch 3,
**dadurch gekennzeichnet, dass** die hypogeale Kammer (1) und die epigeale Kammer (2) ausschließlich durch die Öffnungen, in denen die Wurzelbehälter (5, 6, 7, 8) eingeschlossen sind, in Verbindung miteinander stehen, wobei die hypogeale Kammer (1) und die epigeale Kammer (2) nur durch die Oberfläche des Substrats, welches der Luft des epigealen Kompartiments (2) ausgesetzt ist, in Kontakt miteinander stehen, um hierdurch zu erreichen, dass die Austausche zwischen dem hypogealen Kompartiment (1) und dem epigealen Kompartiment (2) vorteilhafterweise nur über das Substrat zum Wachstum der Pflanzen, wie es auch unter natürlichen Bedingungen geschieht, vermittelt werden.

18. Mikrokosmos nach Anspruch 1,
**dadurch gekennzeichnet, dass** die Systeme zum Beobachten und zum Verwalten der Thermodauer der hypogealen Kammer (1) und der epigealen Kammer (2) die Verwendung von Peltierzellen (10) vorsehen, welche leicht sind, weit entfernt von mühsam, in der Lage sind, sowohl den Schritt der Erwärmung als auch den Schritt der Abkühlung zu bewerkstelligen, und in der Lage sind, auch den Ventilationsschritt zu bewerkstelligen.

19. Mikrokosmos nach einem der vorhergehenden Ansprüche,
**dadurch gekennzeichnet, dass** die hypogeale Kammer (1) auf Rädern (4) befestigt ist.

## Revendications

1. Microcosme pour la croissance de plantes dans un conditionnement biotique et abiotique, pour obtenir un environnement dans lequel reproduire, à l'échelle d'un laboratoire, ce qui se produit dans des conditions naturelles dans une plante ou dans une couverture de plantes lorsque des conditions environnementales données sont imposées et qu'interviennent des organismes qui sont à même d'interagir ou d'interférer avec les fonctions de la plante, ledit microcosme étant constitué par un appareil qui comprend en combinaison :
deux chambres indépendantes l'une de l'autre, dont l'une est une chambre hypogée (1) qui est la partie de l'appareil ou du microcosme qui est conçue pour la croissance de la partie radicale des plantes et de la rhizosphère, et l'autre est une chambre épigée (2) qui est la partie de l'appareil ou du microcosme qui est conçue pour la croissance de la partie aérienne des systèmes végétaux et la phyllosphère, et comprend en outre :
- des moyens pour une gestion active des paramètres microclimatiques qui affectent la vie des plantes et leurs symbiotes ;
- des moyens pour une surveillance passive des paramètres microclimatiques qui affectent la vie des plantes et de leurs symbiotes ;
- des moyens d'acquisition et de traitement de façon constante, continue et méthodique des données relatives aux paramètres micro-environnementaux ; et
- des moyens de logement de sondes de surveillance et de réalisation d'échantillonnages dans le compartiment épigé (2) et le compartiment hypogé (1), réduisant à un minimum la perturbation des conditions micro-environnementales ;
dans lequel la chambre hypogée (1) et la chambre épigée (2) sont assemblées verticalement l'une sur l'autre de façon à constituer un complexe unique et une structure fonctionnellement intégrée,
ledit microcosme étant **caractérisé en ce que** ledit moyen de gestion active, de gestion passive, d'acquisition et de traitement d'une manière constante, continue et méthodique des paramètres environnementaux comprend :
- un système d'alimentation en énergie et de commande générale ;
- un système de surveillance et de gestion active de la thermopériode dans la chambre hypogée (1) ;
- un système de surveillance de l'humidité du substrat racinaire dans la chambre hypogée (1) ;
- un système de surveillance du pH du substrat racinaire dans la chambre hypogée (1) ;
- un système de surveillance du potentiel d'oxydo-réduction du substrat racinaire dans la chambre hypogée (1) ;
- un système de surveillance et de gestion active de la photopériode dans la chambre épigée (2) ;
- un système de surveillance de l'intensité lumineuse dans la chambre épigée (2) ;
- un système de surveillance du spectre lumineux dans la chambre épigée (2) ;
- un système de surveillance et de gestion active de la thermopériode dans la chambre épigée (2) ;
- un système de surveillance et de gestion active de l'humidité relative dans la chambre épigée (2) ;
- un système de surveillance et de gestion active du flux d'air dans la chambre épigée (2) ;
- un système de surveillance du niveau d'humidification de feuille dans la chambre épigée (2) ;
- un système de surveillance et de gestion active de la teneur en dioxyde de carbone dans la chambre épigée (2) ; et
- un système de traitement des données micro-environnementales détectées de façon constante, continue et méthodique dans les deux chambres : la chambre hypogée (1) et la chambre épigée (2).

2. Microcosme selon la revendication précédente, **caractérisé en ce que** ladite chambre hypogée (1) consiste en un compartiment à panneau, doté de panneaux ouvrables (3) pour permettre une inspection interne, une insertion de sondes, des échantillonnages, un retrait et un nettoyage.

3. Microcosme selon la revendication précédente, **caractérisé en ce que** ledit compartiment de la chambre hypogée (1) est équipé pour loger différents types de conteneurs (5, 6, 7, 8) qui doivent contenir le substrat racinaire approprié à différentes architectures de l'appareil radicalaire.

4. Microcosme selon la revendication précédente, **caractérisé en ce que** lesdits conteneurs sont constitués de cylindres (5) et/ou de boîtes (7) et des soucoupes correspondantes (8) et peuvent être en un matériau qui assure un degré élevé de transparence de façon à permettre également une analyse visuelle de la croissance racinaire.

5. Microcosme selon la revendication 2, **caractérisé en ce que** le compartiment constituant la chambre hypogée (1) est thermostaté par ledit système de surveillance et de gestion active de la thermopériode.

6. Microcosme selon la revendication 3, **caractérisé en ce que** des sondes pour surveiller le niveau d'humidité, le pH et le potentiel d'oxydo-réduction du sol sont insérées dans le substrat racinaire.

7. Microcosme selon la revendication 3, **caractérisé en ce que** les conteneurs (5, 6, 7, 8) pour la croissance des racines sont interchangeables grâce au fait qu'ils sont logés dans des guides constitués par des étagères interchangeables (9).

8. Microcosme selon la revendication 3, **caractérisé en ce que** lesdits conteneurs (5, 7) ont des trous dans des positions adéquates et de dimensions telles qu'elles permettent un accès à chaque point du substrat et des racines.

9. Microcosme selon la revendication 1, **caractérisé en ce que** la chambre épigée (2) consiste en un compartiment à panneau, dont la partie structurelle peut être appliquée sur la chambre hypogée (1) et est entourée de quatre panneaux latéraux (3) et d'un panneau supérieur (3), dans lequel lesdits panneaux peuvent être ouverts et retirés pour permettre une inspection interne, une insertion de sondes, des échantillonnages, un retrait et un nettoyage.

10. Microcosme selon la revendication précédente, **caractérisé en ce que** les panneaux latéraux (3) de la chambre épigée (2) et de la chambre hypogée (1) sont remplaçables et peuvent être en un matériau qui assure un degré élevé de transparence, de façon à permettre également une analyse visuelle de la croissance de la partie aérienne et de la partie radicalaire.

11. Microcosme selon la revendication précédente, **caractérisé en ce que**, s'il est nécessaire de réaliser des tests qui imposent l'absence de lumière exogène, lesdits panneaux latéraux (3) peuvent être obscurcis en appliquant simplement des films adhésifs opaques sur la surface.

12. Microcosme selon la revendication 10 ou la revendication 11, **caractérisé en ce que** le compartiment constituant la chambre épigée (2) est doté d'un système de surveillance et de gestion active de la photopériode, de la thermopériode, de l'humidité relative et de la teneur en dioxyde de carbone atmosphérique, ainsi que de capteurs pour la surveillance de l'intensité lumineuse, du spectre lumineux, de la vitesse du flux d'air, et du taux d'humidification de feuille.

13. Microcosme selon la revendication précédente, **caractérisé en ce que** le système de surveillance et de gestion active de la photopériode prévoit un éclairage obtenu avec des lampes capables d'émettre par photosynthèse une lumière active appropriée, selon les besoins expérimentaux, pour stimuler des conditions de croissance végétative, un stimulus reproductif ou les deux.

14. Microcosme selon la revendication 3, **caractérisé en ce que** la dimension en hauteur des conteneurs (5, 7) pour la partie radicalaire des plantes, et en conséquence, de la chambre hypogée entière (1) est conçue en prenant en compte le fait que dans la nature, et en particulier dans un champ cultivé, la partie radicalaire des plantes se développe principalement dans la partie la plus supérieure du sol et principalement dans les premiers 50 à 60 cm de profondeur.

15. Microcosme selon la revendication précédente, **caractérisé en ce que** la dimension en largeur et en profondeur de la chambre hypogée (1) est réalisée en prenant en compte la nécessité d'avoir un nombre minimal de reproductions des conteneurs racinaires pour assurer l'articulation du concept expérimental et la fiabilité des résultats - pas moins de deux reproductions pour chaque type de conteneur - et pour répondre à la nécessité pour chaque point du compartiment d'être atteint facilement par l'opérateur pour l'insertion d'une sonde, des échantillons et le nettoyage du compartiment.

16. Microcosme selon la revendication 9, **caractérisé en ce que** la dimension en hauteur de la chambre épigée (2) est conçue de façon à permettre aux plantes de croître dans le microcosme pour atteindre potentiellement un rapport entre la profondeur de l'appareil radicalaire et la hauteur de la partie aérienne non inférieur à un, limitant ainsi autant que possible tout effet de rabougrissement et de vieillissement précoce des plantes.

17. Microcosme selon la revendication 3, **caractérisé en ce que** la chambre hypogée (1) et la chambre épigée (2) sont en communication l'une avec l'autre exclusivement par les ouvertures dans lesquelles les conteneurs racinaires (5, 6, 7, 8) sont logés, et donc, lorsque ces trous sont mis en prise par les conteneurs racinaires, la chambre hypogée (1) et la chambre épigée (2) sont en contact uniquement par la surface du substrat exposée à l'air du compartiment épigé (2), obtenant ainsi que les échanges entre le compartiment hypogé (1) et le comportement épigé (2) soient avantageusement induits uniquement par le substrat pour la croissance des plantes lorsqu'elle survient dans des conditions naturelles.

18. Microcosme selon la revendication 1, **caractérisé en ce que** lesdits systèmes de surveillance et de gestion active de la thermopériode de la chambre hypogée (1) et de la chambre épigée (2) prévoient l'utilisation de cellules de Peltier (10), qui sont légères, loin d'être encombrantes, capables de gérer à la fois l'étape de chauffage et l'étape de refroidissement, et capable de gérer également l'étape de ventilation.

19. Microcosme selon l'une quelconque des revendications précédentes, **caractérisé en ce que** ladite chambre hypogée (1) est montée sur roues (4).
